# EUROPEAN PATENT SPECIFICATION

(11) **EP 1 455 600 B1**
(45) Date of publication and mention of the grant of the patent: **08.10.2008**
(21) Application number: 02792794.6
(22) Date of filing: 10.12.2002
(51) Int. Cl.: A23L 1/30, A61K 36/73, A61P 19/00

(54) **FOOD OR PET FOOD COMPOSITION CONTAINING PLANT EXTRACT FROM AMELANCHIER FOR BONE HEALTH**
LEBENSMITTEL ODER TIERFUTTER ENTHALTEND DIE KNOCHENGESUNDHEIT UNTERSTÜTZENDES PFLANZENEXTRAKT AUS AMELANCHIER
COMPOSITION ALIMENTAIRE ET COMPOSITION ALIMENTAIRE POUR ANIMAUX CONTENANT UN EXTRAIT VÉGÉTAL D'AMELANCHIER FAVORISANT LA SANTÉ DES OS

(30) Priority: 11.12.2001 EP 01204839
(43) Date of publication of application: 15.09.2004
(73) Proprietor: SOCIETE DES PRODUITS NESTLE S.A., 1800 Vevey (CH)
(72) Inventor: OFFORD-CAVIN, Elizabeth, CH-1041 Poliez-Pittet (CH); FEDERICI, Ermanno, Oxford, OX3 7NP (GB); LEMAURE, Bernard, F-37000 Tours (FR); COURTOIS, Didier, F-37550 St-Avertin (FR)
(74) Representative: Dixon, Sarah
(86) International application number: PCT/EP2002/013174
(87) International publication number: WO 2003/053166

(56) References cited:
- WO-A-01/03716
- WO-A-99/55351
- CN-A- 1 169 863
- DE-A- 19 814 725
- US-A1- 2001 024 664
- DATABASE WPI Section Ch, Week 199836 Derwent Publications Ltd., London, GB; Class D16, AN 1998-426120 XP002198406 & RU 2 102 460 C (UFA WINE WKS), 20 January 1998 (1998-01-20)

## Description

This invention relates to a human or pet composition for maintenance of bone health or prevention, alleviation and/or treatment of bone disorders. It also relates to the use of the composition in the manufacture of a nutritional product, a supplement or a medicament; for promoting bone growth or for the maintenance of bone health.

### Background of the Invention

Healthy bones require effective bone remodeling involving an equilibrium between bone formation and resorption. Most bone diseases are due to increased bone resorption , rendering its inhibition a primary therapeutic objective, therefore most pharmaceutical agents, developed to date, are anti-resorptive. For example, estrogens block production of cytokines that promote osteoclast generation and differentiation. SERMs (selective estrogen response modulator), are being developed which provide benefits for bone health while reducing the risk of adverse hormonal effects on breast or endometrial tissues. It is assumed that they work by a similar mechanism to estrogen in bone. Bisphosphonates (such as alendronate, risedronate etc) concentrate in bone and are, to date, the most effective inhibitors of bone resorption. They inhibit a critical enzyme pathway, required for osteoclast activity and survival. Calcitonin is a polypeptide hormone that inhibits bone resorption by blocking osteoclast activity. New targets include blocking of the TNF receptor/ligand family members and their signalling pathways, particularly of RANK/RANKL, inhibition of bone-specific metalloproteinases such as cathepsin K or inhibition of specific kinases.

Development of therapeutic agents stimulating bone formation has lagged behind that of resorption. Some chemical or pharmaceutical agents are known for promoting bone growth in human. For example, WO 9619246 describes a method for promoting bone growth in a human patient by (normally intermittent not continuous) administration of parathyroid hormone, PTH-related protein or an agonist for at least one month. In WO 9619501, a pancreatic-derived factor inhibits the resorption of bone and stimulates bone cells to proliferate and increases the formation of bone.

DE 198 14 725 A discloses the use of a *Taraxacum* extract for the prevention or treatment of damage to bone marrow (*ie* bone marrow aplasia), and for slowing down bone demineralisation.

US 2001/024664 A1 discloses a method for inhibiting the activity of COX-2 in an organism, the method comprising the step of administering to the organism a composition comprising a therapeutically or prophylactically effective amount of an organic extract of an edible plant, such as *Taraxacum.* Extracts of the claimed invention are useful to treat arthritis, including but not limited to rheumatoid arthritis, spondyloarthopathies, gouty arthritis, osteoarthritis.

WO 99/55351 A discloses a herbal composition, characterized by the fact that it comprises dandelion root (*Teraxaci radix*) at 6-12 wt%. It is disclosed that dandelion is used in popular medicine in case of arthritis and rheumatic troubles.

Although these chemicals and pharmaceutical compounds have been proved for the treatment of bone disorders, it would be of interest to provide a safe, efficient nutritional way to promote bone growth and prevent or alleviate the symptoms of bone/joint disorders in mammals.

### Summary of the Invention

Accordingly, in a first aspect, the present invention provides a food composition in accordance with claim 1.

Remarkably, it has now been found that some plants or plant extracts have a particular positive effect on bone formation and repair, on maintenance of bone health or prevention, alleviation and/or treatment of bone disorders.

The composition according to the invention can be used for the manufacture of a nutritional product, a supplement a treat or a medicament intended for humans or pets.

Administering to a mammal a composition according to the present invention, results in an improved bone regeneration during fracture healing, It helps to inhibit bone resorption. It also helps to increase bone formation and bone mineral density during growth and optimize peak bone mass. Also, this composition helps to decrease bone loss, in particular bone loss associated with age in mammals. It reduces risk of osteoporosis. Furthermore it helps to build cartilage in mammals, to prevent osteoarthritis in pets or humans, which results in a better activity or mobility of the individual.

In another aspect, the invention relates to the use of a plant or plant extract as described above, for the preparation of a composition intended for the prevention, alleviation and/or treatment of bone disorders or maintenance of bone health in mammals.

It also relates to the use of a plant or plant extract as described above, for the preparation of a composition intended for inhibiting bone resorption.

It further relates to the use of a plant or plant extract as described above, for the preparation of a composition intended for improving activity and/or mobility of pets or humans.

The invention further provides the use of a plant or plant extract from Amelanchier for the preparation of a food or pet food composition for increasing bone formation, bone mineral density during growth and optimize peak bone mass, treating or preventing osteoporosis, stimulating bone regenaration during fracture healing which comprises administering an effective amount of a composition as described above.

It further relates to the use of a plant or plant extract from Amelanchier for the preparation of a food or pet food composition for the treatment, alleviation and/or prophylaxis of osteoarthritis in pets or in humans, comprising the step of feeding an individual, a composition as described above.

It further provides a use of a plant or plant extract from Amelanchie for the preparation of a food or pet food composition for decreasing bone loss, in particular bone loss associated with age in humans and pets, comprising the step of feeding an individual, a composition as described above.

### Detailed Description of the Invention

With respect to the fit object of the present invention, the plant or plant extract is from *Amelanchier.*

In a preferred embodiment, the plant or plant extract is *Amelanchier ovalis, Amelanchier alnifolia, Amelanchier laevis, Amelanchier arborea, Amelanchier asiatica,* for example.

The plant according to the invention may be from any part of the plant source, e.g. leaves, tubers, fruit or roots. In a most preferred embodiment, fruits of *Amelanchier species*, are used. The plant or plant extract may be in the form of a dried, lyophilized extract of leaves, roots and/or fruits depending on the source of plant, or fresh plant, or enriched fraction obtained by inorganic or organic solvant extraction process known in the art.

The plant or plant extract according to the invention may be used in the preparation of a food composition. The said composition may be in the form of a nutritionally balanced food or pet food, a dietary supplement, a treat or a pharmaceutical composition.

The plant or plant extract may be used alone or in association with other plants such as chicory, tea, cocoa, or with other bioactive molecule such as antioxidants, fatty acids, prebiotic, fibers, glucosamine, chondroitin sulphate, for example.

In one embodiment, a food composition for human consumption is prepared. This composition may be a nutritional complete formula, a dairy product, a chilled or shelf stable beverage, soup, a dietary supplement, a meal replacement, and a nutritional bar or a confectionery.

Apart from the plant extract according to the invention, the nutritional formula may comprise a source of protein. Dietary proteins are preferably used as a source of protein. The dietary proteins many be any suitable dietary protein; for example animal proteins (such as milk proteins, meat proteins and egg proteins); vegetable proteins (such as soy protein, wheat protein, rice protein, and pea protein); mixtures of free amino acids; or combinations thereof. Milk proteins such as casein, whey proteins and soy proteins are particularly preferred. The composition may also contain a source of carbohydrates and a source of fat.

If the nutritional formula includes a fat source, the fat source preferably provides about 5% to about 55% of the energy of the nutritional formula; for example about 20% to about 50% of the energy. The lipids making up the fat source may be any suitable fat or fat mixtures. Vegetable fats are particularly suitable; for example soy oil, palm oil, coconut oil, safflower oil, sunflower oil, corn oil, canola oil, lecithins, and the like. Animal fats such as milk fats may also be added if desired.

A source of carbohydrate may be added to the nutritional formula. It preferably provides about 40% to about 80% of the energy of the nutritional composition. Any suitable carbohydrates may be used, for example sucrose, lactose, glucose, fructose, corn syrup solids, and maltodextrins, and mixtures thereof. Dietary fibre may also be added if desired. If used, it preferably comprises up to about 5% of the energy of the nutritional formula. The dietary fibre may be from any suitable origin, including for example soy, pea, oat, pectin, guar gum, gum arabic, and fructooligosaccharides. Suitable vitamins and minerals may be included in the nutritional formula in an amount to meet the appropriate guidelines.

One or more food grade emulsifiers may be incorporated into the nutritional formula if desired; for example diacetyl tartaric acid esters of mono- and di- glycerides, lecithin and mono- and di-glycerides. Similarly suitable salts and stabilisers may be included. Vitamins and minerals may also be combined with the plant extract.

The nutritional formula is preferably enterally administrable; for example in the form of a powder, tablet, capsule, a liquid concentrate, solid product or a ready-to-drink beverage. If it is desired to produce a powdered nutritional formula, the homogenised mixture is transferred to a suitable drying apparatus such as a spray drier or freeze drier and converted to powder.

In another embodiment, a nutritional composition comprises a milk based cereal together with a prebiotic formulation. Preferably the milk based cereal is an infant cereal which acts as a carrier for the prebiotic formulation.

In another embodiment, a usual food product may be enriched with at least one plant or plant extract according to the present invention. For example, a fermented milk, a yoghurt, a fresh cheese, a renneted milk, article of confectionery, for example a sweet or sweetened beverage, a confectionery bar, breakfast cereal flakes or bars, drinks, milk powders, soy-based products, non-milk fermented products or nutritional supplements for clinical nutrition.

The amount of the plant or plant extract in the composition may vary according to the plant source and its utilization. In a preferred embodiment, an efficient daily dose amount is of at least about 1 mg, and more preferably from 1 mg to 200mg of the active molecule per day.
In one embodiment, a pharmaceutical composition containing at least an extract or phytochemical as described above, in an amount sufficient to achieve the desired effect in an individual can be prepared. This composition may be a tablet, a liquid, capsules, soft capsules, pastes or pastilles, gums, or drinkable solutions or emulsions a dried oral supplement, a wet oral supplement. The pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated. It will be appreciated that the skilled person will, based on his own knowledge select the appropriate components and galenic form.

The plant or plant extract according to the invention may be used in the preparation of a pet food composition. The said composition may be administered to the pet as a supplement to its normal diet or as a component of a nutritionally complete pet food, and more preferably in an hypocaloric pet food. It may also be a pharmaceutical composition.

The plant or plant extract may be used alone or in association with other plants such as chicory, tea, cocoa, or with other bioactive molecule such as antioxidants, fatty acids, prebiotic fibers, glucosamine, chondroitin sulphate for example.

Preferably, the pet food composition contains about 0.01 to 0.5 g of dry plants per gram of dry pet food for a 15 kg dog; and 0.001 to 0.1 g of dry plants per gram of wet pet food for a 15 kg dog.

The nutritionally complete pet food composition according to the invention may be in powdered, dried form, a treat or a wet, chilled or shelf stable pet food product. It may be chilled or provided as a shelf stable product. These pet foods may be produced by ways known in the art.

The pet food may optionally also contain a prebiotic, a probiotic microorganism or another active agent, for example a long chain fatty acid. The amount of prebiotic in the pet food is preferably less than 10% by weight. For example, the prebiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight.

If a probiotic micro-organism is used, the pet food preferably contains about 10⁴ to about 10¹⁰ cells of the probiotic micro-organism per gram of the pet food; more preferably about 10⁶ to about 10⁸ cells of the probiotic micro-organism per gram. The pet food may contain about 0.5% to about 20% by weight of the mixture of the probiotic micro-organism; preferably about 1% to about 6% by weight; for example about 3% to about 6% by weight.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

In another embodiment, dietary adjuncts may be prepared so as to improve pet food quality. As dietary adjuncts, they may be encapsulated or may be provided in powder form and packaged in conjunction with or separately from a main meal, be it wet or dry. By way of example, a powder containing extracts according to the invention, may be packed in sachets in a powder form or in a gel or lipid or other suitable carrier. These separately packaged units may be provided together with a main meal or in multi-unit packs for use with a main meal or treat, according to user instructions.

The amount of pet food to be consumed by the pet to obtain a beneficial effect will depend on the size of the pet, the type of pet, and age of the pet. However, an amount of the pet food to provide a daily amount of about 0.5 to 5 g of dry plants per kg of body weight, would usually be adequate for dogs and cats.

Administering to a human or animal, the food or pet food composition as described above, results in an improved bone regeneration during fracture healing. It helps to stimulate bone formation and bone mineral density during growth and optimize peak bone mass. In particular it may provide an optimal bone growth during childhood for humans or pets. This food composition helps to prevent bone loss, in particular bone loss associated with age in mammals or bone loss associated with long term hospitalization. It reduces risk of osteoporosis. Furthermore it helps to build cartilage in mammals, prevent osteoarthritis in humans and pets, which results in a better activity or mobility of the individual.

The following examples are given by way of illustration only and in no way should be construed as limiting the subject matter of the present application. All percentages are given by weight unless otherwise indicated. The examples are preceded by a brief description of the figure.

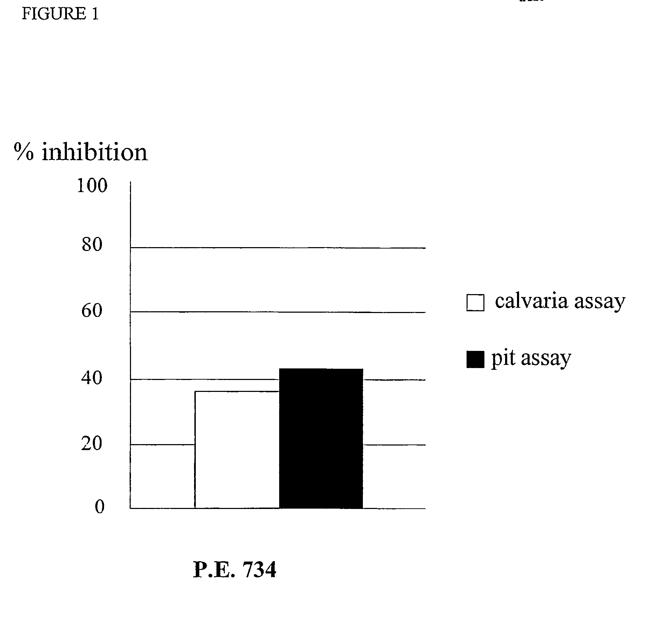

**Figure 1** : comparison of measured inhibition values for the Calvaria Assay (A) and the Pit Assay (B) for the extract of fruits of *Amelanchier alnifolia* (10 µg/ml) : P.E. 734.

### Examples

### Example 1 : Effect of Amelanchier and Taraxacum (comparative) species on bone formation

Extracts of the plant species *Amelanchier* and *Taraxacum* (comparative) were tested for their potential to induce bone formation or inhibit bone resorption

### Materials and methods

### Preparation of extracts for screening assays:

The ground plant material is defatted with hexane then extracted with a mixture of alcohol and water, with different percentages of water from 10 to 90%, preferably with 50%. The alcohols can be methyl or ethyl alcohols, giving the extract 1a.

On an aliquot of the residue of this first extract, an enzymatic hydrolysis is carried out with a and β glucosidases Enzymes can be replaced by acidic conditions. The operation may be done under mild conditions (room temperature) or through reflux with different acid concentrations. The aqueous hydrolysed phase is extracted with a non-miscible solvent, preferably ethylacetate to give the extract 2a.

The extract can be dried, freeze-dried or supplied as a liquid form.
In some cases, polyphenols can be discarded through a polyvinylpolypyrrolidone (PVPP) treatment, avoiding artefact with the screening assays.

Following the extract preparation, each extract was weighed, redissolved in dimethylsulphoxide (DMSO) to a final concentration of 20 mg/ml and stored in aliquots at -20°C. This was used as a stock solution and was subsequently diluted in media for each assay, A range of doses was tested in the assay systems. Subfractions were prepared by fractionation on reverse phase silica gel cartridge with elution by solvents of varying polarity

### • Bone formation assay

BMP-2 luciferase assay-The activity of the extracts was determined using 2T3 cells containing the BMP-2 promoter operatively linked to the luciferase gene. An increase in luciferase activity in cell lysates reflects an increase in BMP-2 promoter activity. The extracts were assayed at an initial dilution of 100 µg/ml with ½ dilutions down to 0.2 µg/ml. BMP-2 promoter activity was measured by measuring the luciferase activity in cell extracts.

Extracts of *Amelanchier ovalis* and *Taraxacum officinalis* (comparative) gave significant positive results in stimulation of BMP-2 expression (Table 1)

| **Latin name** | **English name** | **part** | **conc µg/ml** | **Active extract/n°** |
|---|---|---|---|---|
| *Amelanchier* o*valis* | service berry | fruit | 10 | MeOH/water/219 |
| *Taraxacum officinalis* (comparative) | common dandelion | leaves | 50 | ethylacetate/750/2034 and subfraction 2035 |

Extracts of *Amelanchier ovalis* (P.E. 219 (MeOH/water)), and of *Taraxacum officinale* (comparative) (P.E. 750 (ethylacetate)) were further tested in a human periosteal /preosteoblast cell line, hPOB-tert for their ability to induce the endogenous expression of BMP-2.

The validation of this assay was performed with statins as a positive control. At confluence, cells were incubated with 0.5 µg/ml Lovastatin or with the plant extracts (10-50 µg/ml) for 24h-48h. Total RNA was extracted with TRIzol Reagent (Gibco). 10 µg RNA were reverse transcribed using the 1st Strand cDNA Synthesis Kit (Boehringer). BMP-2 cDNA sequences were amplified for 35 cycles at an annealing temperature of 55°C using specific oligonucleotide primers (5':TTGCGGCTGCTCAGCATGTT; 3':CATCTTGCATCTGTTCTCGGAA). PCR products were separated by agarose gel electrophoresis and detected by ethidium bromide staining. Quantification was performed using NIH Image Software and normalizing results with Actin as house- keeping gene.

### Results :

For *Amelanchier ovalis* (ext conc 10 µg/ml): induction of BMP-2 by 1.5 fold. For *Taraxacum officinale* (comparative) (ext conc 50 µg/ml) induction of BMP-2 by 2.0 fold

### Example 2 : Effect of Amelanchier and Taraxacum (comparative) species on bone resorption

The ability of the extracts prepared as in example 1, to inhibit IL-1 (10⁻¹⁰ M) stimulated bone resorption was assessed in the neonatal bone resorption assay. Each extract was assessed for its capacity to inhibit bone resorption at 10 µg/ml

Extracts of fruits of *Amelanchier alnifolia* (10 µg/ml) (P.E. 734 (ethylacetate)) were able to inhibit IL-1 induced bone resorption in the murine calvaria assay ( R.J. Murills, "In vitro Bone Resorption Assays" in Principles of Bone Biology (Academic Press) 1986, chap. 90). This effect was confirmed in a second bone resorption test, namely the pit assay using rabbit bone mixed cell cultures on bovine bone slices (Tezuka K., et al., 1992, Biochem. Biophys. Res. Commun. 186(2):911-7 and Lorget F., et al., 2000, Biochem. Biophys. Res. Commun. 268(3):899-903). Resorption pits are visualized by staining for TRAP (tartrate resistant acid phosphatase) positive cells and counted.

A comparison of activity of the extracts at 10 µg/ml in the two assay systems is shown in Figure 1.

### Example 3: Dry pet food

A feed mixture is made up of about 58% by weight of corn, about 5.5% by weight of corn gluten, about 22% by weight of chicken meal, 2.5% dried chicory, about 10% of extract of *Taraxacum* leaves (comparative), salts, vitamins and minerals making up the remainder.

The feed mixture is fed into at preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food has a positive effect on bone and cartillage health and increase their mobility.

## Claims

1. A food composition intended for the prevention, alleviation and/or treatment of bone disorders or maintenance of bone health in humans and pets, which comprises as an active ingredient an effective amount of at least one plant or plant extract of *Amelanchier*.

2. A composition according to claim 1, wherein the plant or plant extract is selected from the group consisting of *Amelanchier ovalis, Amelanchier alnifolia, Amelanchier laevis, Amelanchier arborea* and *Amelanchier asiatica*

3. A composition according to claims 1 or 2, wherein the plant or plant extract is from fruits of *Amelanchier species.*

4. A composition according to one of claims 1 to 3, wherein the plant extract is used alone or in association with other plants such as chicory, tea, cocoa, or with other bioactive molecule such as antioxidants, fatty acids, prebiotic fibers, glucosamine or chondroitin sulphate.

5. A composition according to one of claims 1 to 4, which is in the form of a nutritionally balanced food or pet food, a dietary supplement, a treat or a pharmaceutical composition.

6. Use of a plant or a plant extract from *Amelanchier*, for the preparation of a food or pet food composition intended for the prevention, alleviation and/or the treatment of bone disorders or maintenance of bone health in humans or pets.

7. The use of a plant extract from *Amelanchier,* for the preparation of a food or pet food composition intended to help bone regenaration during fracture healing, increase bone formation and bone mineral density during growth and optimize peak bone mass or to decrease bone loss, in particular bone loss associate with age in humans or pets.

8. The use of a plant or a plant extract from *Amelanchier*, for the preparation of a food or pet food composition intended for preventing or alleviating the symptoms of osteoartritis in humans or pets.

9. The use of one of claims 6-8 to improve activity and/or mobility of the individual.

10. The use of one of claims 6-9 to help to build cartilage in humans or pets.

11. The use according to claim 6-10, wherein the composition is as described in one of claims 1 to 5.

## Patentansprüche

1. Nahrungsmittelzusammensetzung, die für die Prävention, Linderung und/oder Behandlung von Knochenleiden oder für die Aufrechterhaltung der Knochengesundheit bei Menschen und Haustieren bestimmt ist, die als aktiven Bestandteil eine wirksame Menge wenigstens einer Pflanze oder eines Pflanzenextrakts von *Amelanchier* umfasst.

2. Zusammensetzung nach Anspruch 1, wobei die Pflanze oder der Pflanzenextrakt aus der Gruppe ausgewählt ist, die besteht aus *Amelanchier ovalis, Amelanchier alnifolia, Amelanchier laevis, Amelanchier arborea* und *Amelanchier asiatica*.

3. Zusammensetzung nach den Ansprüchen 1 oder 2, wobei die Pflanze oder der Pflanzenextrakt aus den Früchten von *Amelanchier* Spezies stammt.

4. Zusammensetzung nach irgendeinem der Ansprüche 1 bis 3, wobei der Pflanzenextrakt allein oder in Assoziation mit anderen Pflanzen verwendet wird, wie beispielsweise Chicorée, Tee, Kakao, oder mit einem anderen bioaktiven Molekül wie Antioxidantien, Fettsäuren, präbiotischen Ballaststoffen, Glucosamin oder Chondroitinsulfat.

5. Zusammensetzung nach irgendeinem der Ansprüche 1 bis 4, die in Form eines ernährungsmäßig ausgewogenen Nahrungsmittels oder Tierfutters, einer diätetischen Ergänzung, eines Happens oder einer pharmazeutischen Zusammensetzung vorliegt.

6. Verwendung einer Pflanze oder eines Pflanzenextrakts von *Amelanchier* zur Herstellung einer Nahrungsmittel- oder Haustierfutterzusammensetzung, die für die Prävention, Linderung und/oder die Behandlung von Knochenleiden oder zur Aufrechterhaltung der Knochengesundheit bei Menschen oder Haustieren bestimmt ist.

7. Verwendung eines Pflanzenextrakts aus *Amelanchier* zur Herstellung einer Nahrungsmittel- oder Haustierfutterzusammensetzung, die dazu bestimmt ist, die Knochenregeneration während der Heilung eines Bruchs zu unterstützen, die Knochenbildung und die Knochenmineraldichte während des Wachstums zu steigern und die Spitzen-Knochenmasse zu optimieren oder einen Knochenverlust zu vermindern, insbesondere einen Knochenverlust, der mit dem Alter von Menschen oder Haustieren assoziiert ist.

8. Verwendung einer Pflanze oder eines Pflanzenextrakts von *Amelanchier* zur Herstellung einer Nahrungsmittel- oder Haustierfutterzusammensetzung, die zur Prävention oder Linderung der Symptome von Osteoarthritis bei Menschen oder Haustieren bestimmt ist.

9. Verwendung nach einem der Ansprüche 6 bis 8 zur Verbesserung der Aktivität und/oder Mobilität der Person.

10. Verwendung nach einem der Ansprüche 6 bis 9 zur Unterstützung der Knorpelbildung bei Menschen oder Haustieren.

11. Verwendung nach Anspruch 6 bis 10, wobei die Zusammensetzung eine ist, wie sie in einem der Ansprüche 1 bis 5 beschrieben wird.

## Revendications

1. Composition alimentaire destinée à la prévention, au soulagement et/ou au traitement de troubles osseux ou au maintien de la santé osseuse chez les êtres humains et les animaux de compagnie, qui comprend comme ingrédient actif une quantité efficace d'au moins une plante *Amelanchier* ou d'au moins un extrait de plante *Amelanchier.*

2. Composition suivant la revendication 1, dans laquelle la plante ou l'extrait de plante est choisi dans le groupe consistant en *Amelanchier ovalis, Amelanchier alnifolia, Amelanchier laevis, Amelanchier arborea* et *Amelanchier asiatica.*

3. Composition suivant la revendication 1 ou 2, dans laquelle la plante ou l'extrait de plante provient des fruits d'une espèce d*'Amelanchier.*

4. Composition suivant l'une des revendications 1 à 3, dans laquelle l'extrait de plante est utilisé seul ou en association avec d'autres plantes telles que la chicorée, le thé, le cacao, ou avec une autre molécule biologiquement active telle que des antioxydants, des acides gras, des fibres prébiotiques, la glucosamine ou le sulfate de chondroïtine.

5. Composition suivant l'une des revendications 1 à 4, qui est sous forme d'un aliment ou aliment pour animaux de compagnie nutritionnellement équilibré, d'un supplément diététique, d'une friandise ou d'une composition pharmaceutique.

6. Utilisation d'une plante ou d'un extrait de plante *Amelanchier,* pour la préparation d'un aliment ou d'une composition alimentaire pour animaux de compagnie, destiné à la prévention, au soulagement et/ou au traitement de troubles osseux ou au maintien de la santé osseuse chez les êtres humains ou les animaux de compagnie.

7. Utilisation d'un extrait de plante *Amelanchier,* pour la préparation d'un aliment ou d'une composition alimentaire pour animaux de compagnie destiné à permettre une régénération osseuse au cours de la guérison de fractures, une augmentation de la formation de tissus osseux et de la densité minérale osseuse au cours de la croissance et une optimisation de la masse osseuse maximale ou une diminution de la perte de tissu osseux, en particulier de la perte de tissu osseux associée à l'âge chez les êtres humains ou les animaux de compagnie.

8. Utilisation d'une plante ou d'un extrait de plante *Amelanchier,* pour la préparation d'un aliment ou d'une composition alimentaire pour animaux de compagnie, destiné à la prévention ou au soulagement des symptômes d'arthrose chez les êtres humains ou les animaux de compagnie.

9. Utilisation suivant l'une des revendications 6 à 8 pour améliorer l'activité et/ou la mobilité de l'individu.

10. Utilisation suivant l'une des revendications 6 à 9 pour permettre la formation de cartilage chez les êtres humains ou les animaux de compagnie.

11. Utilisation suivant les revendications 6 à 10, dans laquelle la composition est telle que décrite dans l'une des revendications 1 à 5.
